(19) Europäisches Patentamt
European Patent Office
Office européen des brevets

(11) Veröffentlichungsnummer: **0 176 070 B1**

(12) # EUROPÄISCHE PATENTSCHRIFT

(45) Veröffentlichungstag der Patentschrift: **19.04.89**

(51) Int. Cl.⁴: **C 07 K 5/10**, C 07 K 7/06, A 61 K 37/02

(21) Anmeldenummer: **85112028.7**

(22) Anmeldetag: **23.09.85**

(54) **Pharmakologisch aktive peptide.**

(30) Priorität: **28.09.84 DE 3435727**

(43) Veröffentlichungstag der Anmeldung: **02.04.86 Patentblatt 86/14**

(45) Bekanntmachung des Hinweises auf die Patenterteilung: **19.04.89 Patentblatt 89/16**

(84) Benannte Vertragsstaaten: **AT BE CH DE FR GB IT LI LU NL SE**

(56) Entgegenhaltungen:
**EP-A-0 019 829**
**EP-A-0 053 029**

**CHEMICAL ABSTRACTS, Band 102, 1985, Seite 465, Nr. 110584a, Columbus, Ohio, US; V. BRANTL: "Novel opioid peptides derived from human beta-casein: human beta-casomorphins", & EUR. J. PHARMACOL. 1984, 106(1), 213-14**
**CHEMICAL ABSTRACTS, Band 102, 1985, Seite 76, Nr. 125725w, Columbus, Ohio, US; M. YOSHIKAWA et al.: "Opioid peptides from human beta-casein", & AGRIC. BIOL. CHEM. 1984, 48(12), 3185-7**

(73) Patentinhaber: **Brantl, Victor, Dr. med., Dipl.-Chem., Hermann- Burte- Strasse 14, D-7846 Schliengen (DE)**

(72) Erfinder: **Brantl, Victor, Dr. med., Dipl.- Chem., Hermann- Burte- Strasse 14, D-7846 Schliengen (DE)**

(74) Vertreter: **Schacht, Wilhelm, Dr. jur., Am Eselsweg 47, D-6500 Mainz 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

## Beschreibung

Die Erfindung betrifft pharmakologisch aktive Peptide insbesondere opiatartig wirkend.

Aufgabe der Erfindung ist es, neue, bisher noch nicht beschriebene Peptide zu schaffen, die insbesondere opiatartig wirken.

Diese Aufgabe ist gemäß der Erfindung dadurch gelöst, daß die Peptide folgende Struktur aufweisen:

Tyr-A-Phe-Val-T
Tyr-A-Phe-Val-B-T
Tyr-A-Phe-Val-B-Pro-T
Tyr-A-Phe-Val-B-Pro-C-T
Tyr-A-Phe-Val-B-Pro-C-Pro-T
Tyr-A-Phe-Val-B-Pro-C-Pro-D-T

wobei Tyr gleich der Aminosäure Tyrosin ist und das N-terminale L-Tyrosin der allgemeinen Formel:

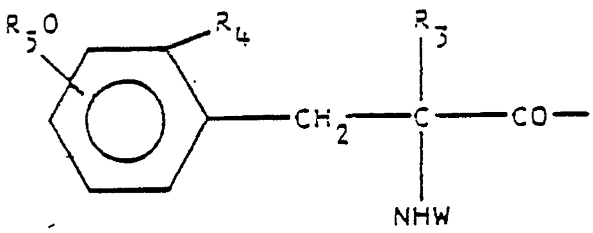

vorliegt, wobei im einzelnen steht:

$R_3$ für Wasserstoff oder eine Alkylgruppe mit 1 bis 4 C-Atomen,

$R_4$ für Wasserstoff oder zusammen mit $R_3$ für eine Äthylbrücke,

$R_5$ für Wasserstoff, eine Alkylgruppe mit 1 bis 4 C-Atomen oder eine $R_6CO$-Gruppe,

$R_6$ für einen gesättigten oder ungesättigten, geradkettigen oder verzweigten Alkylrest mit 1 bis 17 C-Atomen, einen Phenylrest oder elnen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy- mit 1 bis 4 C-Atomen substituiert sein können, wobei die $R_5O$-Gruppe sich in meta- oder para-Stellung zum

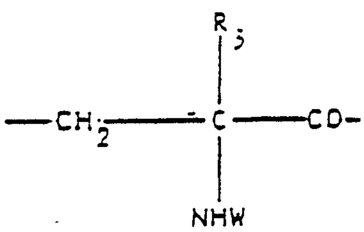

-Rest befIndet,

W für Wasserastoff Alkyl mit 1 bis 5 C-Atomen Alkenyl mit 3 bis 5 C-Atomen, Cyclopropylmethyl, Cyclobutylmethyl, $R_6CO$-, H-Val, H-Arg, H-Lys, H-Ile, H-Tyr oder H-Phe.

Phe gleich der Aminosäure Phenylalanin ist, das auch in der allgemeinen Formel vorliegt:

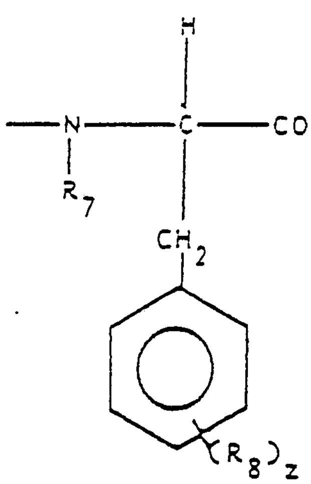

wobei im einzelnen bedeuten:

$R_7$ für Wasserstoff oder Alkyl mlt 1 bis 4 C-Atomen,

$R_8$ für Wasserstoff, Fluor, Chlor, Brom, Nitro, Alkyl mit 1 bis 4 C-Atomen, Z für 1 oder 2 steht.

Val gleich der Aminosäure Valin ist, Pro gleich der Aminosäure Prolin ist, das auch in der allgemeinen Formel vorliegt:

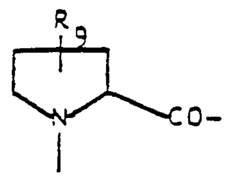

wobei im einzelnen bedeuten:

$R_9$ = Wasserstoff, eine Hydroxygruppe, eine Alkyl- oder Alkoxygruppe mit 1 oder 4 C-Atomen - am Stickstoff eine Alkyl- oder Alkoxygruppe mit 1 bis 4 C-Atomen gebunden ist - eine oder mehrere Ketogruppen in den Ring eingeführt sind;

A gleich L-Prolin oder eine beliebige D Aminosäure ist; B, C und D können jede beliebige Aminosäure der D- oder L-form sein. T steht für OH, OR, $NH_2$, NHR, $NR_2$ oder NHNHR', wobei R gegebenenfalls folgende Bedeutung hat: Substit. lineares oder verzweigtes $C_{1-10}$-Alkyl, Adamantyl $C_{1-10}$-Cycloalkyl oder $C_{6-8}$-Aralkyl, zweckmäßigerweise Phenyl, Benzyl oder Phenyläthyl bedeutet und R' Wasserstoff, lineares oder verzweigtes $C_{1-10}$-Alkyl, Cycloalkyl oder $C_{6-8}$-Aralkyl, lineares, verzweigtes oder cyclisches aliphat. $C_{1-16}$-Acyl, gegebenenfalls durch OH, $NH_2$, $C_{1-14}$-Alkoxy oder Halogen substituiert, aromatisches Acyl, gegebenenfalls durch OH, $NH_2$, Halogen oder $C_{1-4}$-Alkoxy substituiert; lineares verzweigtes oder cyclisches $C_{3-11}$aliphatisches Urethan darstellt und deren pharmazeutische annehmbare Salze.

Nach einer weiteren Ausbildung sind dle Peptide dadurch gekennzeichnet, daß A= L-Prolin, B= L-Glutaminsäure, C = L-Isoleucin und D = L-Tyrosin ist.

Weiterhin ist es besonders vorteilhaft, wenn A = D-Alanin, D-Threonin, D-Serin, D-Methionin, D-Valin, D-Phenylalanin, D-Leucin, D-Isoleucin, D-Arginin, D-Histamin, D-Prolin, D- Hydroxyprolin, D-Lysin, D-Glutamin, D-Glutaminsäure, D-Asparagin, D-Asparaginsäure, B = L-Glutaminsäure, L-Tyrosin oder L-Phenylalanin, C = L-Isoleucin und D = L-Tyrosin ist.

Die D-Aminosäure an Position 2 des Peptids, vom N-terminalen Ende aus gerechnet, bewirkt eine erhebliche Steigerung der opiatartigen Wirkung, verbunden mit einer Stabilisierung der Peptide gegen Abbau durch proteolytische Enzyme im Blut.

Nach einer anderen Weiterbildung der Erfindung liegen die Aminosäuren Prolin Phenylalanin sowie B, C und D als De- hydroaminosäuren vor.

Nach einer besonders vorteilhaften Weiterbildung der Erfindung ist das Phenylalanin in der 3. Aminosäureposition des Peptids (vom N-terminalen Ende des Peptids her gerechnet) in der D-Form vorliegend.

Dies hat insbesondere den Vorteil, daß die opiatartige Wirkung erhalten bleibt und eine besonders hohe Stabilität gegenüber peptidspaltenden Enzymen resultiert, da ja Peptidbindungen, an denen D-Aminosäuren beteiligt sind, nur schwer von den natürlichen Proteasen abgebaut werden.

Den gleichen, o. g. stabilisierenden Effekt weisen die Peptide auf, wenn das Valin in der 4. Aminosäureposition in der D-Form vorliegt.

Die erfindungsgemäßen Peptide können starke Wirkungen auf das Zentrale Nervensystem ausüben.

Diese Wirkung kann z. B. nach intracerebroventrikulärer Gabe der Peptide an Ratten beobachtet werden. Die Tiere zeigen einen katatonen Zustand (Körperstarre), wie dies u. a. mit Neuroileptika-Wirkungen vergleichbar ist.

Weiterhin können die erfindungsgemäßen Peptide endokrine Wirkungen auslösen.

Dies kann insbesondere eine Wachstumshormonspiegelerhöhung (GH) oder eine insulinfreisetzende Wirkung sein.

Die erfindungsgemäßen Peptide lösen in vivo und in vitro opiatartige Wirkungen aus.

Die gemäß der Erfindung hergestellten Peptide können blutdrucksenkende Wirkungen in vivo auslösen.

Besonders vorteilhaft sind insbesondere Peptide folgender Struktur:

Tyr-Pro-Phe-Val-OH
Tyr-Pro-Phe-Val-$NH_2$
Tyr-Pro-Phe-Val-Glu-OH
Tyr-Pro-Phe-Val-Glu-Pro-OH
Tyr-Pro-Phe-Val-Glu-Pro-Ile-OH
Tyr-Pro-Phe-Val-Glu-Pro-Ile-Pro-OH
Tyr-Pro-Phe-Val-Glu-Pro-Ile-Pro-Tyr-OH
Tyr-D-Ala-Phe-Val-OH
Tyr-D-Ala-Phe-Val-$NH_2$
Tyr-D-Ala-Phe-Val-Glu-OH
Tyr-D-Ala-Phe-Val-Tyr-OH
Tyr-D-Ala-Phe-D-Val-Tyr-OH

Tyr-D-Ala-Phe-D-Val-Tyr-$NH_2$
Tyr-D-Ala-Phe-D-Val-Phe-$NH_2$
Tyr-D-Ala-Phe-D-Val-Phe-OH

Die erfindungsgemäßen Peptide können nach Kopplung an Thyreoglobulin oder andere makromolekulare Eiweißkörper als Antigene zur Erzeugung von Antikörpern im Säugetierorganismus verwendet werden.

Hierbei wird das Peptid in üblicher Weise mit Carbodiimid an das Makromolekül gekoppelt und intracutan in die Rücken und die Bauchhaut von Kaninchen injiziert.

Die so gewonnenen Antikörper können dann zur Bestimmung der erfindungsgemäßen Peptide in Körpergeweben und -Flüssigkeiten verwendet werden.

Dies kann sowohl zur Bestimmung von exogen zugeführten, wie auch endogen anstandener Peptide verwendet werden (Diagnostik).

Die erfindungsgemäßen Peptide und/oder deren Derivate und/oder deren Salze können in Human- und Tierarzneimitteln enthalten sein.

Diese können insbesondere als Antitussiva, Antidiarrhoika, Analgetika, Antipsychotika, Tranquilizer Verwendung finden. Die Peptide können auch zur Wachstumsförderung als Tierfuttermitteladditive Verwendung finden.

**Biespiel**

Synthese zweier erfindungsgemäßer Peptide folgender Sequenz:
Tyr-Pro-Phe-Val-OMet/-OH
Tyr-Pro-Phe-Val-Glu-OMet/-OH

Die verwendete Synthesemethode benützt Benzyloxycarbonyl-geschützte Aminosäuren als gemischte Anhydride (Lottspeich et al., Hoppe-Seyler's Z. Physiol. Chem., 1835 - 1839, 1980).

Hierbei wird die Aminokomponente bei -15°C oder niedriger in Dimethylformamid (DMF) mit einem 0.5 molaren Überschuß an gemischten Anhydrid einer Z-Aminosäureisobutylcarbonsäure (wobei Z- als Schutzgruppe dient und einen N-Benzyloxycarbonyl-Rest darstellt) 2 - 4 Stunden umgesetzt. Nach der 2 - 4 stündigen Kupplung wird der Überschuß an Anhydrid zerstört. Bei 0°C wird dann der pH-Wert des Reaktionsproduktes mit wässriger, gesättigter $KHCO_3$-Lösung auf 8 eingestellt und 30 Minuten bei 0°C gerührt.

Die Peptide werden mit Äthylacetat extrahiert. Das Äthylacetat/Peptidgemisch wird, um das Z-Aminosäure-Kaliumsalz zu entfernen, 3 mal mit NaCL/Wasser, 3 mal mit Wasser gewaschen und abgedampft. Das so erhaltene Peptid, welches noch die Schutzgruppe Z trägt, wird dann in Methanol hydriert. Hierbei werden 100 - 500 mg Pd/Aktivkohle als Katalysator pro mmol Peptid zugesetzt. Die $CO_2$-Abspaltung wird mit $Ba(OH)_2$-Lösung kontrolliert. Der Katalysator wird dann abfiltriert (Papierfilter), mit Wasser ausgiebig gewaschen und das Filtrat auf einem ationsverdampfer eingedampft. Das gewünschte deblockierte Peptid ist im Rückstand enthalten.

I. Herstellung des Zwischenproduktes Phe-Val-Glu-OMet (I)

I/Stufe 1 a) Herstellung des gemischten Anhydrids Z-Phe-Val-gem. Anhydrid.

603 mg (1.5 mmol, = 50 %-iger Überschuß) des Dipeptids Z-L-Phe-L-Val (Bachem, Schweiz) werden in 20 ml DMF mit 200 µl (1.5 mmol) Chlorameisensäureisobutylester bei -15°C nach Zusatz von 160 µl (1.5 mmol) N-Methylmorpholin 15 Minuten umgesetzt.

I/Stufe 1 b) Vorbereitung der Aminokomponente.

223 mg (1.0 mmol) L-Glutaminsäure-α,γ-dimethylester. HCl werden in 20 ml DMF unter Zusatz von 110 µl (1.0 mmol) N-Methylmorpholin bei -15°C aufgelöst.

I/Stufe 2 Umsetzung des gem. Anhydrids der Stufe I/1a mit der Aminokomponente der Stufe I/1b.

Das Z-Phe-Val-gem. Anhydrid wird mit 1 b) in insgesamt 40 ml DMF bei -15°C 4 Stunden zum Z-Phe-Val-Glu-α,γ-dimethylester umgesetzt.

Vor der Aufarbeitung wird der 50 %-ige Überschuß an gemischtem Anhydrid zerstört. Bei 0°C wird der ph-Wert des Reaktionsproduktes mit wässriger, gesättigter $KHCO_3$-Lösung auf pH 8 eingestellt und 30 Minuten bei 0°C gerührt. Danach wird das Peptid mit 50 - 100 ml Äthylacetat (EtAc) extrahiert; das EtAc/Peptidgemisch wird mit gesättigter, wässriger NaCl-Lösung gewaschen. Nach einem weiteren abschließenden Waschen mit Wasser wird die EtAc-Phase eingedampft.

I/Stufe 5 Abspalten der Schutzgruppe durch Hydrierung.

Das Peptid wird in 30 ml Methanol gelöst und 100 mg Palladium auf Aktivkohle (Merck) zugegeben. Nach dem Verdrängen der Luft durch Stickstoff wird in das Reaktionsgefäß Wasserstoff eingeleitet. Die Hydrierung wird bei 25° - 30°C durchgeführt. Die Hydrierung ist beendet, wenn kein $CO_2$ mehr freigesetzt wird, d.h. wenn nach Kontrolle in wässriger $Ba(OH)_2$-Lösung kein Niederschlag mehr gebildet wird. Die Lösung wird filtriert, mit Wasser gewaschen und am Rotationsverdampfer einrotiert. Das verbleibende Produkt wird dann später als Aminokomponente eingesetzt.

II. Herstellung des Zwischenproduktes Phe-Val-OMet (II)

II/Stufe 1 a) Herstellung des gemischen Anhydrids.

447 mg (1.5 mmol) Z-L-Phe werden in 20 ml DMF unter Zusatz von 170 µl (1.5 mmol) N-Methylmorpholin

gelöst und mit 180 µl Chlorameisensäureisobutylester bei -15° C 15 Minuten umgesetzt.

II/Stufe 1 b) Vorbereitung der Aminokomponente.

168 mg (1.0 mmol) L-Valin-Methylester. HCL werden in 20 mi DMF unter Zusatz von 170 µl (1.5 mmol) N-Methylmorpholin gelöst.

II/Stufe 2 Umsetzung des gem. Anhydrids der Stufe II/1a mit der Aminokomponente der Stufe II/1a mit der Aminokomponente der Stufe II/1b.

Das Z-L-Phe-gem Anh. wird in insgesamt 40 ml DMF bei -15° C mit der Aminokomponente II/1b zum Z-L-Phe-L-Val-OMet umgesetzt.

Die Aufarbeitung erfolgt wie bereits unter I/2 und I/3 beschrieben wurde.

III. Weiterverarbeitung der Zwischenprodukte Phe-Val-Glu-OMet (I) und Phe-Val-OMet (II)

III/Stufe 1 a) Herstellung des gem. Anhydrids Z-Pro-gem. Anh.

374 mg (1.5 mmol) Z-L-Prolin werden in 20 ml DMF unter Zusatz von 170 µl (1.5 mmol) N-Methylmorpholin gelöst und mit 200 µl (1.5 mmol) Chlorameisensäureisobutylester bei -15° C 15 Minuten umgesetzt.

III/Stufe 1 b) Als Aminokomponenten werden nunmehr die Zwischenprodukte I und II in 20 ml DMF eingesetzt, wobei jeweils ein Ansatz der Stufe III/1 a) verwendet wird.

Z-L-Pro $\overset{ah}{+}$ L-Phe-L-Val-L-Glu-OMet $\xrightarrow[4\,Std]{-15°C}$ Z-L-Pro-L-Phe-L-Val-L-Glu-OMet

und

Z-L-Pro $\overset{ah}{+}$ L-Phe-L-Val-OMet $\xrightarrow[4\,Std]{-15°C}$ Z-L-Pro-L-Phe-L-Val-OMet

III/Stufe 2 Die Aufarbeitung erfolgt wie bereits oben beschrieben und führt zu den Produkten:

Pro-Phe-Val-Glu-OMet (III a)

Pro-Phe-Val-OMet (III b)

IV. Weiterverarbeitung der Produkte III a und III b

IV/Stufe 1 a) Bildung des gemischten Anhydrids Z-Tyr-gem. Anh.

630 mg (1.4 mmol) N,O-di-Z-Tyrosin werden in 15 ml DMF unter Zusatz von 165 µl (1.4 mmol) N-Methylmorpholin gelöst und mit 175 µl Chlorameisensäureisobutylester bei -15° C 15 Minuten umgesetzt.

IV/Stufe 1 b) Die Zwischenprodukte III a und III b werden jeweils in 15 mi DMF gelöst und werden mit je einem Ansatz IV/Stufe 1a) bei -15° C 4 Stunden umgesetzt.

IV/Stufe 2 Die Zerstörung des gemischten Anhydrids, die Extraktion und die Hydrierung erfolgen wie bereits oben beschrieben.

Die Syntheseendprodukte:

Tyr-Pro-Phe-Val-OMet und

Tyr-Pro-Phe-Val-Glu-$\alpha,\gamma$-diMet

werden dann entweder direkt über Säulenchromatographie (Biogel P 2) gereinigt oder einer sauren Esterhydrolyse unterworfen und analysiert, so wie von Lottspeich et al. (Hoppe-Seyler's Z. Physiol. Chem., 361, 1835 - 1839, 1980) beschrieben. Die gereinigten und mittels Aminosäureanalyse geprüften Peptide:

Tyr-Pro-Phe-Val-OH

Tyr-Pro-Phe-Val-Glu-OH

wurden dann auf pharmakologische Wirkungen hin untersucht.

Die Herstellung der anderen erfindungsgemäßen Peptide erfolgte auf die gleiche Weise, wie dies oben bereits beschrieben wurde, wobei die entsprechend der gewünschten Sequenz andere Z-Aminosäurederivate (z. B. Z-D-Alanin oder Z-D-Valin statt dem oben beschriebenen Z-L-Prolin) bzw. Aminosäureester (Prolin-Methylester, Isoleucin-OMet, Tyrosin-OMet und Phenylalanin-OMet) eingesetzt werden. Die Peptidester können dann zum Zwecke der C-terminalen Amidierung in üblicher Weise einer Ammonolyse unterworfen werden.

**Beispiel**

Pharmakologische Wirkungen zweier erfindungsgemäßer Peptide (Tyr-Pro-Phe-Val-OH und Tyr-Pro-Phe-Pro-Val-Glu-OH).

Die beiden Substanzen zeigen spezifische opiatartige Wirkungen am elektrisch stimulierten plexus myentericus/Längsmuskel-Präparat des Meerschweinchenileums (GPI), Methode nach Schulz und Goldstein, J. Pharmacol. Exptl. Ther. 183, 400, 1972.

Die Ergebnisse sind in Tabelle 1 aufgeführt.

| Substanz | GPI |
|---|---|
| Tyr-Pro-Phe-Val | 56.2 |
| Tyr-Pro-Phe-Val-Glu | 33.0 |
| Normophin | 0.1 |

Tabelle 1. Opiatartige Wirkungen zweier erfindungsgemäßer Peptide; die Zahlen geben diejenige Konzentration (µM) an, die notwendig ist, die elektrisch induzierte Kontraktion des Meerschweinchen-Darm-Präparates (GPI) um 50 % zu hemmen ($IC_{50}$-Wert). Die Zahlen sind Mittelwerte aus 4 Bestimmungen; die Standardabweichung von den Mittelwerten ist kleiner als 12 %. Die Hemmungen des GPI sind mit dem spezifischen Opioid-Antagonisten Naloxon aufhebbar bzw. bei Vorbehandlung blockierbar.

Die entsprechenden D-$Ala^2$-Verbindungen zeigen noch stärkere opiatartige Wirkungen am GPI-Präparat.

Opiatartige Wirkungen können auch nach intracerebroventrikulärer Injektion bei Ratten beobachtet werden. Hierbei wird so vorgegangen, wie von Brantl et al., Life Sciences, 28, 1903 - 1909, 1981 beschrieben wurde. Als wirksame analgetische Dosis erwiesen sich 350 bis 500 µg der o. g. Peptide; die analgetische Wirkung ist mit Naloxon 10 mg pro kg Körpergewicht (intraperitoneal) aufhebbar bzw. bei Vorbehandlung mit Naloxon blockierbar.

**Patentansprüche**

1. Pharmakologisch aktive Peptide der Formel
Tyr-A-Phe-Val-T
Tyr-A-Phe-Val-B-T
Tyr-A-Phe-Val-B-Pro-T
Tyr-A-Phe-Val-B-Pro-C-T
Tyr-A-Phe-Val-B-Pro-C-Pro-T
Tyr-A-Phe-Val-B-Pro-C-Pro-D-T

wobei Tyr gleich der Aminosäure Tyrosin ist und das N-terminale L-Tyrosin der allgemeinen Formel:

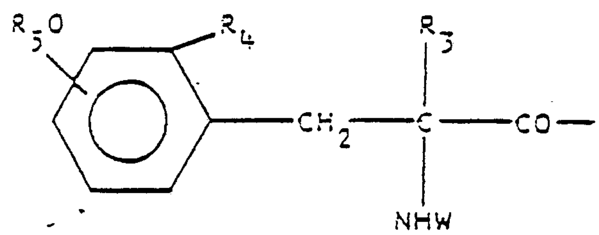

vorliegt, wobei im einzelnen steht:
$R_3$ für Wasserstoff oder eine Alkylgruppe mit 1 bis 4 C-Atomen,
$R_4$ für Wasserstoff oder zusammen mit $R_3$ für eine Äthylbrücke,
$R_5$ für Wasserstoff eine Alkylgruppe mit 1 bis 4 C-Atomen oder eine $R_6CO$-Gruppe,
$R_6$ für einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei dei $R_5O$-Gruppe sich in meta- oder para-Stellung zum
oder para-Stellung zum

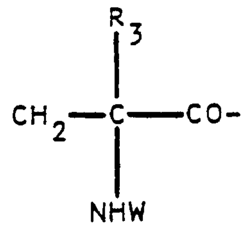
Rest befindet,

W für Wasserstoff Alkyl mit 1 bis 5 C-Atomen, Alkenyl mit 3 bis 5 C-Atomen Cyclopropylmethyl, Cyclobutylmethyl, $R_6CO$-, H-Val, H-Arg, H-Lys, H-Ile, H-Tyr, oder H-Phe.

b) das Phenylalanin der allgemeinen Formel vorliegt:

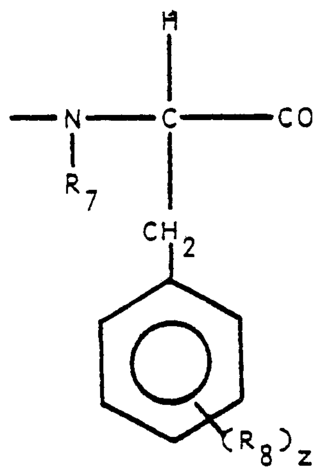

wobei im einzelnen begeuten.

$R_7$ für Wasserstoff oder Alkyl mit 1 bis 4 C-Atomen,

$R_8$ für Wasserstoff, Fluor, Chlor, Brom, Nitro, Alkyl mit 1 bis 4 C-Atomen, z für 1 oder 2 steht.

c) Val gleich der Aminosäure Valin ist:

d) das Prolin der allgemeinen Formel vorliegt:

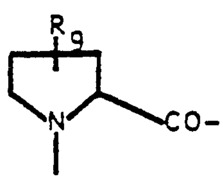

wobei im einzelnen bedeuten:

$R_9$ = Wasserstoff, eine Hydroxygruppe elne Alkyl- oder Alkoxygruppe mit 1 oder 4 C-Atomen - am Stickstoff eine Alkyl- oder Alkoxygruppe mit 1 bis 4 C-Atomen gebunden ist - eine oder mehrere Ketogruppen in den Ring eingeführt sind;

A gleich L-Prolin oder einen beliebige D-Aminosäure ist;

B, C und D können jede beliebige Aminosäure der D- oder L-Form sein.

T steht für OH, OR, $NH_2$, NHR, $NR_2$ oder NHNHR', wobei R gegebenenfalls die folgende Bedeutung hat: Substit. lineares oder verzweigtes $C_{1\text{-}10}$-Alkyl, zweckmäßigerweise Phenyl, Benzyl oder Phenyläthyl bedeutet und R' Wasserstoff, lineares oder verzweigtes $C_{1\text{-}10}$-Alkyl, Cycloalkyl oder $C_{6\text{-}8}$-Arylalkyl, lineares, verzweigtes oder cyclisches aliphat. $C_{1\text{-}16}$-Acyl; gegebenenfalls durch OH, $NH_2$, $C_{1\text{-}14}$-Alkoxy oder Halogen substituiert, aromatisches Acyl, gegebenenfalls durch OH, $NH_2$, Halogen oder $C_{1\text{-}4}$-Alkoxy substituiert; lineares verzweigtes oder cyclisches $C_{3\text{-}11}$aliphatisches Urethan darstellt

und deren pharazeutische annehmbare Salze.

2. Pharmakologisch aktive Peptide nach Anspruch 1, dadurch gekennzeichnet, daß

A = L-Prolin (L-Pro)

B = L-Glutaminsäure (L-Glu)

C = L-Isoleucin (L-Ile)

D = L-Tyrosin (L-Tyr) ist.

3. Pharmakologisch aktive Peptide nach Anspruch 1 und 2, dadurch gekennzeichnet, daß

A = D-Alanin, D-Threonin, D-Serin, D-Methionin, D-Valin, D-Phenylalanin, D-Leucin, D-Isoleucin, D-Arginin, D-Histamin, D-Prolin, D-Hydroxyprolin, D-Lysin, D-Glutamin, D-Glutaminsäure, D-Aspargin oder D-Asparginsäure
B = L-Glutaminsäure, L-Tyrosin oder L-Phenylalanin
C = L-Isoleucin
D = L-Tyrosin ist.

4. Pharmakologisch aktive Peptide nach Anspruch 1 - 3, dadurch gekennzeichnet, daß die Aminosäuren Prolin, Phenylalanin sowie B, C und D als Dehydroaminosäuren vorliegen.

5. Pharmakologisch aktive Peptide nach Anspruch 1 - 4, dadurch gekennzeichnet, daß das Phenylalanin in der 3. Aminosäureposition des Peptids (vom N-terminalen Ende gerechnet) in der D-Form vorliegt.

6. Pharmakologisch aktive Peptide nach Anspruch 1 - 5, dadurch gekennzeichnet, daß das Valin in der 4. Position in der D-Form vorliegt.

7. Pharmakologisch aktive Peptide nach Anspruch 1 - 6, dadurch gekennzeichnet, daß diese Wirkungen auf das zentrale Nervensystem ausüben.

8. Pharmakologisch aktive Peptide nach Anspruch 1 - 7, dadurch gekennzeichnet, daß diese endokrine Wirkungen auslösen.

9. Pharmakologisch aktive Peptide nach Anspruch 1 - 8, dadurch gekennzeichnet daß diese opiatartige Wirkungen auslösen.

10. Pharmakologisch aktive Peptide nach Anspruch 1 - 9, dadurch gekennzeichnet daß diese Wirkungen auf das Herz- und Kreislaufsystem ausüben.

11. Pharmakologisch aktive Peptide nach Anspruch 1 - 10, dadurch gekennzeichnet, daß die Peptide folgende Struktur aufweisen:
Tyr-Pro-Phe-Val-OH
Tyr-Pro-Phe-Val-$NH_2$
Tyr-Pro-Phe-Val-Glu-OH
Tyr-Pro-Phe-Val-Glu-Pro-OH
Tyr-Pro-Phe-Val-Glu-Pro-Ile-OH
Tyr-Pro-Phe-Val-Glu-Pro-Ile-Pro-OH
Tyr-Pro-Phe-Val-Glu-Pre-Ile-Pro-Tyr-OH
Tyr-D-Ala-Phe-Val-OH
Tyr-D-Ala-Phe-Val-$NH_2$
Tyr-D-Ala-Phe-Val-Glu-OH
Tyr-D-Ala-Phe-Val-Tyr-OH
Tyr-D-Ala-Phe-D-Val-Tyr-OH
Tyr-D-Ala-Phe-D-Val-Tyr-$NH_2$
Tyr-D-Ala-Phe-D-Val-Phe-$NH_2$
Tyr-D-Ala-Phe-D-Val-Phe-OH.

12. Pharmakologisch aktive Peptide nach Anspruch 1 - 11, dadurch gekennzeichnet, daß diese nach Kopplung an Thyreoglobulin oder andere makromolekulare Eiweißkörper als Antigene zur Erzeugung von Antikörpern im Säugetierorganismus verwendet werden.

13. Pharmakologisch aktive Peptide nach Anspruch 1 - 12, dadurch gekennzeichnet, daß die Antikörper gegen die erfindungsgemäßen Peptide zur Bestimmung derselben in Körpergeweben und -Flüssigkeiten verwendet werden.

14. Arzneimittel und Tierarzneimittel, dadurch gekennzeichnet, daß diese die in den Ansprüchen 1 - 11 charakterisierten pharmakologisch aktiven Peptide und/oder deren Derivate und/oder deren Säureadditionssalze und/oder deren Metallkomplexe enthalten.

## Claims

1. Pharmacologically active peptides of the formula:
Tyr-A-Phe-Val-T
Tyr-A-Phe-Val-B-T
Tyr-A-Phe-Val-B-Pro-T
Tyr-A-Phe-Val-B-Pro-C-T
Tyr-A-Phe-Val-B-Pro-C-Pro-T
Tyr-A-Phe-Val-B-Pro-C-Pro-D-T
wherein Tyr is equal to the amino acid tyrosine and the N-terminal L-tyrosine of the general formula:

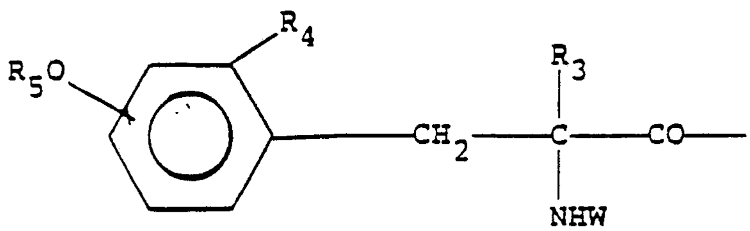

is present wherein the following individually signify:

$R_3$ represents hydrogen or an alkyl group with 1 to 4 C-atoms,

$R_4$ represents hydrogen or together with $R_3$ an ethyl bond,

$R_5$ represents hydrogen, an alkyl group with 1 to 4 C-atoms or a $R_6CO$ group,

$R_6$ represents a saturated or unsaturated, straight chain or branched chain alkyl residue with 1 to 17 C-atoms, a phenyl residue or a phenylalkyl residue with 7 to 12 C-atoms, whereby the phenyl residues can be substituted by 1 or 2 substituents from the halogen series, alkyl with 1 to 4 C-atoms or alkoxy with 1 to 4 C-atoms, whereby the $R_5O$ group is in the meta or para position to

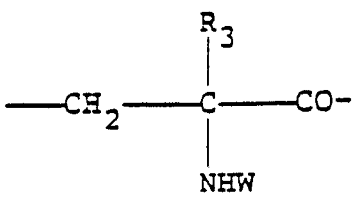 residue

in which W represents hydrogen, alkyl with 1 to 5 C-atoms, alkenyl with 3 to 5 C-atoms, cyclopropylmethyl, cyclobutylmethyl, $R_6CO$-, H-Val, H-Arg, H-Cys, H-Ile, H-Tyr or H-Phe;

b) in which phenylalanine of the general formula is present:

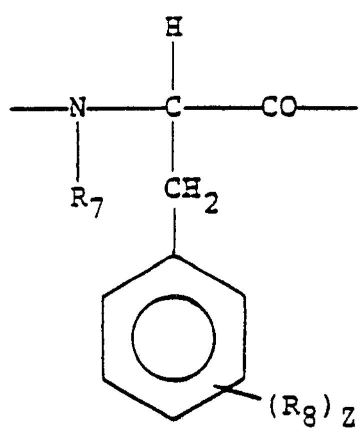

in which:

$R_7$ represents hydrogen or alkyl with 1 to 4 C-atoms,

$R_8$ represents hydrogen, fluorine, chlorine, bromine, nitro, alkyl with 1 to 4 C-atoms, Z stand for 1 or 2.

c) Val represents the amino acid valine,

d) in which the proline of the general formula is present:

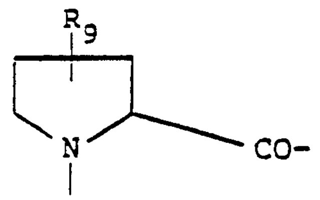

wherein:

$R_9$ represents hydrogen, a hydroxy group, an alkyl - or alkoxy group with 1 or 4 C-atoms
- at the nitrogen, an alkyl or alkoxy group with 1 to 4 C-atoms is combined
- one or several keto groups are introduced into the ring,

A is equal to L-proline or any D-amino acid,
B, C und D can be any amino acid of the D- or L-form;
T represents OH, OR, $NH_2$, NHR, $NR_2$, or NHNHR', wherein R, if necessary, has the following significance: substituting linear or branched $C_{1-10}$-alkyl, adamantyl, $C_{1-10}$-cycloalkyl or $C_{6-8}$-aralkyl, preferably signifies phenyl, benzyl or phenylethyl and R' signifies hydrogen, linear or branched $C_{1-10}$-alkyl, cycloalkyl or $C_{6-8}$-aralkyl, linear, branched or cyclic aliphat. $C_{1-16}$-acyl, substituted, if necessary, by OH, $NH_2$, $C_{1-14}$-alkoxy or halogen; aromatic acyl substituted, if necessary, by OH, $NH_2$, halogen or $C_{1-4}$-alkoxy; linear, branched or cyclic $C_{3-11}$aliphatic urethane and their pharmaceutically acceptable salts.

2. Pharmacologically active peptides according to claim 1, characterized in that:
A = L-Proline (L-Pro)
B = L-Glutamic acid (L-Glu)
C = L-Isoleucine (L-Ile)
D = L-Tyrosine (L-Tyr).

3. Pharmacologically active peptides according to claims 1 and 2, characterized in that:
A = D-Alanine, D-Threonine, D-Serine, D-Methionine, D-Valine, D-Phenylalanine, D-Leucine, D-Isoleucine, D-Arginine, D-Histamine, D-Proline, D-Hydroxyproline, D-Lysine, D-Glutamine, D-Glutamic acid, D-Asparagine or D-Asparaginic acid,
B = L-Glutamic acid, L-Tyrosine or L-Phenylalanine,
C = L-Isoleucine
D = L-Tyrosine.

4. Pharmacologically active peptides according to claims 1 to 3, characterized in that the amino acids proline, phenylalanine, as well as B, C and D are present as de-hydroamino acids.

5. Pharmacologically active peptides according to claims 1 to 4, characterized in that the phenylalanine in the third aminoacid position of the peptide (calculated from the N-terminal and of the peptide) is present in the D-form.

6. Pharmacologically active peptides according to claims 1 to 5, characterized in that the valine in the fourth position in the D-form is present.

7. Pharmacologically active peptides according to claims 1 to 6, characterized in that they effekt the central nervous system.

8. Pharmacologically active peptides according to claims 1 to 7, characterized in that they trigger endocrine effects.

9. Pharmacologically active peptides according to claims 1 to 8, characterized in that they trigger opiate - like effekts.

10. Pharmacologically active peptides according to claims 1 to 9, characterized in that they effekt the heart and the circulatory system.

11. Pharmacologically active peptides according to claims 1 to 10 characterized in that the peptides have the following structure:

Tyr-Pro-Phe-Val-OH
Tyr-Pro-Phe-Val-$NH_2$
Tyr-Pro-Phe-Val-Glu-OH
Tyr-Pro-Phe-Val-Glu-Pro-OH
Tyr-Pro-Phe-Val-Glu-Pro-Ile-OH
Tyr-Pro-Phe-Val-Glu-Pro-Ile-Pro-OH
Tyr-Pro-Phe-Val-Glu-Pro-Ile-Pro-Tyr-OH
Tyr-D-Ala-Phe-Val-OH
Tyr-D-Ala-Phe-Val-$NH_2$
Tyr-D-Ala-Phe-Val-Glu-OH
Tyr-D-Ala-Phe-Val-Tyr-OH
Tyr-D-Ala-Phe-D-Val-Tyr-OH
Tyr-D-Ala-Phe-D-Val-Tyr-$NH_2$
Tyr-D-Ala-Phe-D-Val-Phe-$NH_2$

Tyr-D-Ala-Phe-D-Val-Phe-OH.

12. Pharmacologically active peptides according to claims 1 to 11, characterized in that they can be used as antigens for the production of antibodies in mammalian organism or other macromolecular albumins.

13. Pharmacologically active peptides according to claims 1 to 12, characterized in that the antibodies are used against the peptides according to the invention for the analysis of the same in body tissues and body fluids.

14. Human and animal medication characterized in that these contain pharmacologically active peptides, and/or their derivatives, and/or their acid addition salts, and/or their metal complexes, as characterized in the claims 1 to 11.

**Revendications**

1. Peptides à activité pharmacologique des formules suivantes:
Tyr-A-Phe-Val-T
Tyr-A-Phe-Val-B-T
Tyr-A-Phe-Val-B-Pro-T
Tyr-A-Phe-Val-B-Pro-C-T
Tyr-A-Phe-Val-B-Pro-C-Pro-T
Tyr-A-Phe-Val-B-Pro-C-Pro-D-T
dans lesquelles:
L-Tyr est le résidu d'amino-acide L-tyrosine et la L-tyrosine N-terminale de formule générale est present

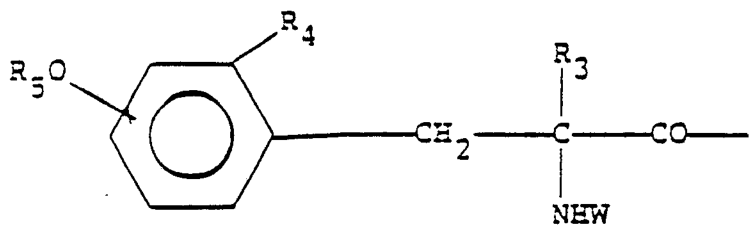

En ce formule générale représente:
$R_3$ un atome d'hydrogène ou un groupe alkyle en $C_1$ à $C_4$,
$R_4$ un atome d'hydrogène ou forme avec $R_3$ un pont éthylène,
$R_5$ un atome d'hydrogène, un groupe alkyle en $C_1$ à $C_4$ ou un groupe $R_6CO$,
$R_6$ un résidu d'alkyle saturé ou non saturé, linéaire ou ramifié comportant 1 à 17 atomes de carbone, un reste phényle ou phénylalkyle comportant 7 à 12 atomes de carbone, les restes phényles pouvant être substutués par 1 ou 2 substituants de la série des halogènes, des restes alkyles en $C_1$ à $C_4$ ou d'alcoxy en $C_1$ à $C_4$, le groupe $R_5O$ étant en position méta- ou para- par rapport au reste

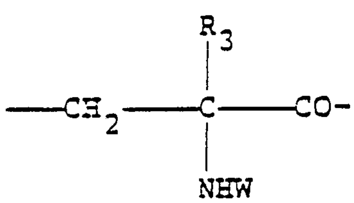

et
W étant un atome d'hydrogène, un alkyle $C_1$ à $C_5$, un cyclopropylméthyle, un cyclobutylméthyle, ou un groupe $R_6CO$-,
b) la phénylalanine est de formule générale:

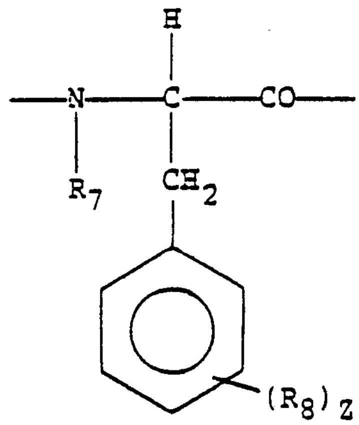

dans laquelle:

$R_7$ est un atome d'hydrogène ou un groupe alkyle en $C_1$ à $C_4$,

$R_8$ est un atome d'hydrogène, -de fluore, -de chlore, -de brome, un groupe nitro, un groupe alkyle en $C_1$ à $C_4$ et,

Z représente 1 ou 2 substituents,

c) Val représente le résidu d'amino acide valine,

d) la proline de formule générale:

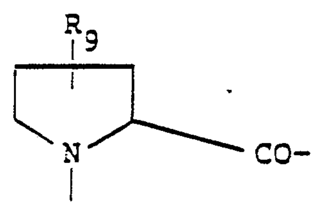

est present.

En ce formule générale représente:

$R_9$ un atome d'hydrogène, un groupement hydroxy ou alcoxy en $C_{1-4}$,

- à nitrogène est attaché un groupe d'alkyle ou un groupe d'alcoxy en $C_1$ à $C_4$,
- un groupe ou plusieurs groupes de Keto sont introduits dans le rond,

A est L-proline ou un résidu d'amino acide quelquonque de la configuration stéréochimique D,

B, C et D péuvent être chaques quelconques résidus d'amino acides de la configuration stéréochimique D ou L.

T est OH, OR, $NH_2$, NHR, $NR_2$ ou NHNHR' dans lesquels R, si necessaire, est:

- un alkyle $C_{1-10}$, adamantyle qui sont linéaires ou ramifiés et substitués;
- un cycloalkyle $C_{1-10}$ ou un aralkyle $C_{6-8}$, avantageusement un phényle, benzyle ou phenylethyle; et R' est
- un atome d'hydrogène,
- un alkyle $C_{1-10}$ linéaire ou ramifié,
- un cycloalkyle ou un aralkyle $C_{6-8}$,
- un groupement d'acyle en $C_{1-16}$ aliphatique, linéaire, ramifié ou cyclique, si necessaire, substitué par OH, $NH_2$, $C_{1-14}$-alcoxy ou halogène,
- acyle aromatique, si necessaire, substitué par OH, $NH_2$, halogène ou alcoxy $C_{1-4}$,
- un groupe de type uréthane linéaire, ramifié ou cyclique $C_{3-11}$ aliphatique,

et

les sels pharmaceutiquement acceptables des substances que l'ont vient de mentionner.

2. Peptides à activité pharmacologique selon la revendication 1, caractérisés en ce que

A représente le résidu d'amino acide L-proline (L-Pro),

B représente le résidu d'amino acide d'acide de L-glutamine (L-Glu),

C représente le résidu d'amino acide L-isoleucine (L-Ile),

D représente le résidu d'amino acide L-tyrosine (L-Tyr).

3. Peptides à activité pharmacologique suivant l'une des revendications 1 et 2, caractérisés en ce que:

A représente les résidus d'amino acides D-alanine, D-threonine, D-serine, D-methionine, D-valine, D-phenylalanine, D-leucine, D-isoleucine, D-arginine, D-histamine, D-proline, D-hydroxyproline, D-lysine, D-glutamine, acide de D-glutamine, D-asparagine ou acide de D-asparagine,

B représente acide de L-glutamine, L-tyrosine ou L-phénylanine,

C représente L-isoleucine,
D représente L-tyrosine.

4. Peptides à activité pharmacologique, suivant l'une des revendications 1 - 3, caractérisés en ce que les amino acides proline, phénylalanine et B, C et D sont dehydro-amino-acides.

5. Peptides à activité pharmacologique, suivant l'une des revendications 1 - 4, caractérisés en ce que le phénylalanine en position troisième d'amino acide du peptide (calculé de bout N-terminal) est présent dans la configuration stéréochimique D.

6. Peptides à activité pharmacologique, suivant l'une des revendications 1 - 5, caractérisés en ce que le valine en position quatrième d'amino acide du peptide (calculé de bout N-terminal) est présent dans la configuration stéréochimique D.

7. Peptides à activité pharmacologique, suivant l'une des revendications 1 - 6, caractérisés en ce qu'ils faient des effets en système nerveux central.

8. Peptides à activitè pharmacologique, suivant l'une des revendications 1 - 7, caractérisés en ce qu'ils faient des effets endocrines.

9. Peptides à activité pharmacologique, suivant l'une des revendications 1 - 8, caractérisés en ce que les peptides montrent des effets opiacés.

10. Peptides à activité pharmacologique, suivant l'une des revendications 1 - 9, caractérisés en ce qu'ils montrent des effets en système cardiovasculaire.

11. Peptides à activité pharmacologique, suivant l'une des revendications 1 - 10, caractérisés en ce que les peptides ont des structures suivantes:
Tyr-Pro-Phe-Val-OH
Tyr-Pro-Phe-Val-$NH_2$
Tyr-Pro-Phe-Val-Glu-OH
Tyr-Pro-Phe-Val-Glu-Pro-OH
Tyr-Pro-Phe-Val-Glu-Pro-Ile-OH
Tyr-Pro-Phe-Val-Glu-Pro-Ile-Pro-OH
Tyr-Pro-Phe-Val-Glu-Pro-Ile-Pro-Tyr-OH
Tyr-D-Ala-Phe-Val-OH
Tyr-D-Ala-Phe-Val-$NH_2$
Tyr-D-Ala-Phe-Val-Glu-OH
Tyr-D-Ala-Phe-Val-Tyr-OH
Tyr-D-Ala-Phe-D-Val-Tyr-OH
Tyr-D-Ala-Phe-D-Val-Tyr-$NH_2$
Tyr-D-Ala-Phe-D-Val-Phe-$NH_2$
Tyr-D-Ala-Phe-D-Val-Phe-OH.

12. Peptides à activité pharmacologique suivant l'une des revendications 1 - 11, caractérisés en ce qu'ils peuvent appliqués comme antigines - après couplage à thyreoglobuline ou à autres corps d'albumine macromoleculaires - à procréation des anticorps dans l'organisme de mammifère.

13. Peptides à activité pharmacologique, suivant l'une des revendications 1 - 12, caractérisés en ce que les anticorps peuvent applicés contre des peptides inventives à determiner les mêmes en tissus de corps et humeurs.

14. Préparations pharmaceutiques, caractérisées en ce qu'elles contiennent des peptides à activité pharmacologique et/ou leurs dérivés et/ou leurs sels d'acide et/ou leurs complexes métallique qu'ils sont definés en revendications 1 - 11.